Europäisches Patentamt

European Patent Office

Office européen des brevets

(11) Publication number: **0 516 873 A1**

# EUROPEAN PATENT APPLICATION

(21) Application number: **91109282.3**

(22) Date of filing: **06.06.91**

(51) Int. Cl.5: **C07B 59/00**, **A61K 49/02**

(43) Date of publication of application:
**09.12.92 Bulletin 92/50**

(84) Designated Contracting States:
**AT BE CH DE DK ES FR GB GR IT LI LU NL SE**

(71) Applicant: **THE STATE of ISRAEL Atomic Energy Commission Soreq Nuclear Research Center Nahal Soreq Yavne 70 600(IL)**

(72) Inventor: **Lavie, Efraim**
**18 Meltzer St.**
**Rehovot 76285(IL)**
Inventor: **Bitton, Mordechi**
**1317/5 Rotem St.**
**Ashdod(IL)**

(74) Representative: **Vossius & Partner**
**Siebertstrasse 4 P.O. Box 86 07 67**
**W-8000 München 86(DE)**

(54) A method and kit for protein labelling with 99 mTC.

(57) Labelling of glycoproteins other than fibrinogen with $^{99m}$Tc. The method comprises:

a) dispersing the glycoprotein in an aqueous basic buffer solution of pH 9.0-9.7;

b) incubating the resulting dispersion with a reducing agent at a temperature lower than the denaturation temperature of the glycoprotein; and

c) treating the resulting product with an aqueous pertechnetate ($^{99m}$Tc) solution to yield the desired $^{99m}$Tc labeled glycoprotein.

The present invention is in the field of isotopic labeling of proteins for radiodiagnostic imaging. More specifically, it relates to the labeling of various proteins with the technetium isotope $^{99m}$Tc which is the meta stable form of $^{99}$Tc.

For radiodiagnostic imaging using proteins specific to certain target organs, various radionuclides may be considered, the most commonly encountered being $^{131}$I(iodine), $^{125}$I, $^{123}$I, $^{111}$In (indium) and $^{99m}$Tc (Technetium). Iodination methods offer the most straight-forward techniques. Among the radio-isotopes of iodine, $^{123}$I nearly meets best the practical requirements but its high cost restricts its usefulness. The main disadvantage of other radio-isotopes of iodine is the deiodination of the protein *in vivo*. $^{111}$In may label proteins via a bifunctional bridge, but usually the In-labeled protein accumulates to a large extent in the liver. In addition, its high cost ($50-$100 per dose) is a serious limitation.

$^{99m}$Tc is considered the most important radionuclide in nuclear medicine due to its six hours half-life and its 140 KeV gamma radiation. For these reasons most gamma cameras are calibrated to Tc photon range. The lower cost of $^{99m}$Tc, (less than $6.00 per dose), and the fact that it is generator-available (while $^{111}$In for example is produced in a cyclotron and is not readily available) are added incentives for the use of $^{99m}$Tc for protein labeling.

Antibodies to tumor-associated antigens have been considered as the leading candidate for protein labeling. Radiolabeled antibodies can be designed and used to detect tumor sites. Successful targeting of diagnostic radionuclide to tumors not only provides a tool to diagnose and stage cancer but also demonstrates the tumor's feasibility for therapy. Monoclonal antibodies have been labeled with $^{99m}$Tc both directly and through bifunctional chelating agents, but with suboptimal results. Technetium-99m labeling of antibodies suffers from reduced immunoreactivity, formation and binding of colloids to the antibody, lack of control of the oxidation level of Tc, and low yields of specifically bound $^{99m}$Tc. Fritzberg et al. [Proc. Natl. Acad. Sci. USA, 85,4025-4029 (88)] have applied a diamide dithiolate ligand system ($N_2S_2$) to $^{99m}$Tc labeling of antibody fragments. This approach led to a high yield of labeling, good stability and retained immunoreactivity. However, their procedure seems tedious and involves several steps, including: preforming the technetium complex, chemical conversion of the carboxylate groups to an active ester, and finally conjugation of the preformed complex to the antibody.

In addition, several methods of labeling proteins with $^{99m}$Tc all of which use a pertechnetate salt for reaction with the protein have already been developed. The main drawback of the reported methods is that they rely upon the protein itself to stabilize the reduced technetium isotope to prevent both the reoxidation thereof to pertechnetate and the formation of radiocolloids and other absorbed forms of $^{99m}$Tc. Stabilization of the technetium isotope by the proteins was found unsuccessful, judging by the reported *in vivo* instabilities of Tc-labeled proteins. Some attempts have been made to stabilize the technetium-protein complex by attaching strong metal chelators, such as diethylenetriamine pentaacetic acid (DTPA), to the protein. However, this approach was found inefficient, owing to the formation of dimers and aggregates of the protein, and to a decrease in the biological activity of the protein. A further disadvantage of this labeling process is its complicated procedure which involves, inter alia, a tedious purification step.

Rhodes et al [Nuc. Med. 21, p.54 (1980)] describe the labeling process of immunoglobulin-G with $^{99m}$Tc involving the incubation of the protein with stannous ions in an acidic buffer solution under nitrogen atmosphere.

Lavie et al. [Int. J. Appl. Radiat. Isot., 35, 2 (1984)] have reported the labeling of fibrinogen with $^{99m}$Tc. According to that process fibrinogen is incubated with stannous chloride in basic buffer solution (pH = 9.8) which is followed by the introduction of pertechnetate ($^{99m}$TcO$_4^-$) into the solution. Since the protein family includes an enormous variety of molecules differing from each other in their sequence of amino acids which form their primary structure, and in their secondary and tertiary structures, it was not to be expected that proteins other than fibrinogen would be amenable to $^{99m}$Tc labeling by a similar method. Indeed, in spite of the long-felt need for the labeling of other proteins with $^{99m}$Tc in order to obtain materials suitable for radiodiagnostic imaging, no such method was available to date.

In accordance with the present invention it has surprisingly been found that glycoproteins in general may be labeled by a procedure similar to the $^{99m}$Tc isotopic labeling of fibrinogen disclosed in the above Lavie et al. publication. Typical examples of proteins that are amenable to labeling in accordance with the present invention are the enzyme tissue plasminogen activator (t-PA), polyclonal immunoglobulin-G (IgG) and avidin.

In accordance with the present invention there is thus provided a method for labeling glycoproteins other than fibrinogen with $^{99m}$Tc, comprising the steps of:

    a) dispersing the glycoprotein in an aqueous basic buffer solution of pH 9.0-9.7;

    b) incubating the resulting dispersion with a reducing agent at a temperature lower than the denaturation temperature of the glycoprotein; and

c) treating the resulting product with an aqueous pertechnetate ($^{99m}$Tc) solution to yield the desired $^{99m}$Tc labeled glycoprotein.

For buffering the aqueous glycoprotein dispersion at a pH of from 9.0-9.7 as stipulated in a) above, any phsyiologically compatible buffering system may be used. Of particular advantage are buffer systems based on glycine and alanine because of their suitability for *in vivo* application.

In step b) any compatible reducing agent capable of reducing the technetium from the heptavalent state in which it is present in the pertechnetate form to the lower valency in which it is bound to the glycoprotein may be used, the preferred reducing agent used in accordance with the invention being stannous chloride ($SnCl_2$).

Suitable pertechnetates of $^{99m}$Tc that can be used in accordance with the invention in step c) above are for example $NH_4TcO_4$($^{99m}$Tc) and $KTcO_4$($^{99m}$Tc).

Since proteins are denatured at elevated temperatures, the incubation temperature in step b) above should be kept at a level at which no denaturation occurs while at the same time being inducive of an interaction of the glycoprotein with the reducing agent. It has been found in accordance with the invention that temperatures within the range of about 15°C to about 40°C are suitable and that in many cases an incubation temperature of about 37°C is most suitable.

It has been found in accordance with the present invention that the reactivity of the glycoproteins is not damaged by the buffered basic solution used in the performance of the $^{99m}$Tc labeling and that it does not affect the blood clearance pattern.

Because of the six hour half-life time of technetium, it is preferred that the actual labeling operation be carried out only shortly before the labeled glycoprotein is required using for the purpose a pertechnetate ($^{99m}$Tc) prepared from freshly obtained $^{99m}$Tc. Accordingly, in a preferred embodiment of the invention the labeling procedure is carried out in two stages, in the first of which only the above steps a) and b) are performed and the product thereof is stored and in the second stage the above step c) is performed.

Where prolonged storage periods are envisaged the product of the first stage is preferably lyophilized to produce a glycoprotein impregnated with reducing agent and a buffer agent, and which product is stored until performance of the second stage.

For the performance of the above preferred embodiment the invention further provides a kit comprising at least one vessel holding a lyophilized glycoprotein impregnated with a reducing agent and a buffer agent as obtained in steps a) and b) above.

In accordance with one embodiment of such kit the said impregnated glycoprotein is held in a plurality of separate vessels each containing a unit dosage. If desired, two or more glycoproteins may be included within one kit.

The shelf life of the lyophilized, impregnated glycoproteins in a kit according to the invention is at least six months and may be as long as three years or even more.

The $^{99m}$Tc labeling process of glycoproteins in accordance with the invention was found to be applicable to glycoproteins in general regardless of structure and configuration. Nor is the nature of the glycoprotein labelled with $^{99m}$Tc in accordance with the invention dependent on its biological properties as demonstrated, for example, by t-PA, immunoglobulin-G and avidin all of which are glycoproteins that are amenable to $^{99m}$Tc labeling in accordance with the present invention. Thus, t-PA is a glycoprotein enzyme used as a drug for treating patients with acute myocardial infarction; immunoglobulin is a defense glycoprotein (antibody) serving in the immune system of vertebrates; and avidin is a tetramer glycoprotein (mw-66,000) that exists in egg yolk and is known to be useful for many *in vitro* diagnostic applications due to its high affinity to biotin. These three proteins differ from one another and all of them differ radically from fibrinogen which is the labeling substrate in the above Lavie et al. publication. Thus, none of them has the same or even similar amino acid sequence as fibrinogen and the sizes, charges, spatial arrangements and biological properties are also different. Furthermore, while $t\frac{1}{2}$ of the t-PA blood clearance pattern is of the order of 1-2 minutes, the $t\frac{1}{2}$ of the blood clearance pattern of fibrinogen is about 48-72 hours.

The invention will now be specifically described in the following examples, it being understood that it is not limited thereto.

Example 1: Tissue plasminogen activator (t-PA)

t-PA solution (1mg/ml) was concentrated to 4 mg/ml by ultrafiltration. The protein solution containing arginine was replaced by dyalysis against 0.15M alanine buffer at pH 9.5 containing 0.01% of the detergent Tween-80 (trademark). The enzyme solution was incubated at 37°c with a reducing agent: $SnF_2$ or $SnCl_2$ (0.02 mg) for 5h under a nitrogen atmosphere. Then the pertechnetate solution was added and the resulting solution was let to stand for 45 min. The labeling yield was 65% determined by thin layer chromatography, precipitation in 10% trichloroacetic acid and separation in gel permeation columns. Fibrin-plate assay showed that labeled t-PA had the same lysing potential as unlabeled t-PA.

Example 2: Labeling of inhibited-t-PA

t-PA was inhibited by treating the protein with diisopropylfluorophosphate (DFP) and then labeled with Tc. The labeling procedure was as for the native t-PA in Example 1 and led to exactly the same labeling result.

Example 3: The human polyclonal antibody immunoglobulin-G (Ig G)

3-5 mg of the IgG were dyalized against an amino acid buffer (glycine) at pH>9.0. The solution was incubated at room temperature with SnCl$_2$ solution (final amount 0.05mg) for 3h under a nitrogen atmosphere. 1-2 ml of TcO$_4$$^-$ in saline was added ($^{99m}$Tc activity was 2mC). Labeling yield was $^-$80%.

Example 4: Preparation of IgG kit

A protein-stannous chloride solution - prepared according to Example 3 was lyophilized according to the following procedure: 20 h incubation at 5°C, 2 h at 15°C, and finally 2 h at 30°C. The exact composition of the kit was: IgG, 3.8 mg; Sodium citrate, 2.6 mg; Stannous chloride, 0.1 mg; Glycine, 3.7 mg; and final pH 9.1. This preparation led to 90% labeling yield of the detected solution.

The SDS-PAGE (gel electrophoesis) runs of the kit, with and without the addition of Tc, proved that the chemical and labeling modification of the IgG molecule did not affect the molecular weight of the antibody. The kit was stable for six months.

Example 5: Labeling of Avidin

5 mg/ml of avidin were dissolved in glycine buffer, pH-9.6. The protein solution was incubated with SnCl$_2$ (0.1 mg) for 7h under a nitrogen atmosphere. Then 0.5 ml of TcO$_4$$^-$ in saline was added and the resulting solution was allowed to stand for 1h at room temperature. The labeling yield was 62%. The affinity of the labeled avidin to biotin was found to be close to that of unlabeled avidin.

**Claims**

1. A method for labeling glycoproteins other than fibrinogen with $^{99m}$Tc, comprising the steps of:
   a) dispersing the glycoprotein in an aqueous basic buffer solution of pH 9.0-9.7;
   b) incubating the resulting dispersion with a reducing agent at a temperature lower than the denaturation temperature of the glycoprotein; and
   c) treating the resulting product with an aqueous pertechnetate ($^{99m}$Tc) solution to yield the desired $^{99m}$Tc labeled glycoprotein.

2. A method according to claim 1 or 2, wherein the buffer in said aqueous basic buffer solution is based on glycine or alanine.

3. A method according to claim 1 or 2, wherein stannous chloride is used as reducing agent.

4. A method according to any one of claims 1 to 3, wherein the incubation temperature in step b) is from about 15°C to about 40°C.

5. A method according to claim 4, wherein the incubation temperature is about 37°C.

6. A method according to any of claims 1 to 5 wherein in a first stage only steps a) and b) are performed, the product of these steps is stored and step c) is performed in a second stage.

7. A method according to claim 6, wherein the said product of steps a) and b) is lyophilized to produce a glycoprotein impregnated with reducing agent and a buffer agent, which product is stored until performance of the second stage.

8. A kit for the performance of the method of claim 7, comprising at least one vessel holding a lyophylized glycoprotein impregnatead with a reducing agent and a buffer agent.

9. A kit according to claim 8 comprising a plurality of vessels each holding a unit dosage of the lyophylized glycoprotein.

10. A kit according to claim 8 or 9 comprising at least two different lyophylized glycoproteins.

))) European Patent Office

# EUROPEAN SEARCH REPORT

Application Number

EP    91 10 9282

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 403 225 (IMMUNOMEDICS) <br> * page 6, column 9, line 57 - column 10, line 48; claims; examples * | 1-10 | C07B59/00 <br> A61K49/02 |
| A | EP-A-0 005 638 (SERONO LABORATORIES) <br> * page 9, line 9 - page 10, line 11; claims; example II * | 1-10 | |
| D,A | INTERNATIONAL JOURNAL OF APPLIED RADIATION AND ISOTOPES. <br> vol. 35, no. 2, 1984, OXFORD GB <br> pages 99 - 102; <br> E. LAVIE ET AL: 'FACTORS AFFECTING THE LABELING OF HUMAN FIBRINOGEN WITH 99m-Tc IN VITRO' <br> * the whole document * | 1-10 | |
| D,A | JOURNAL OF NUCLEAR MEDECINE. <br> vol. 21, no. 6, June 1980, NEW YORK US <br> B. A. RHODES ET AL: 'A KIT FOR DIRECT LABELING OF ANTIBODIES AND ANTIBODY FRAGMENTS WITH Tc-99m' <br> PROCEEDINGS OF THE 27th ANNUAL MEETING <br> * page P54 - page P55 * | 1-10 | TECHNICAL FIELDS SEARCHED (Int. Cl.5) <br><br> C07B <br> A61K |

The present search report has been drawn up for all claims

| Place of search | Date of completion of the search | Examiner |
|---|---|---|
| THE HAGUE | 17 FEBRUARY 1992 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)